# EUROPÄISCHE PATENTSCHRIFT

(11) **EP 3 060 270 B1**
(45) Veröffentlichungstag und Bekanntmachung des Hinweises auf die Patenterteilung: **19.04.2017**
(21) Anmeldenummer: 14786920.0
(22) Anmeldetag: 21.10.2014
(51) Int. Cl.: A61M 1/10

(54) **VERFAHREN ZUM BETRIEB EINER PUMPENEINRICHTUNG SOWIE PUMPENEINRICHTUNG**
METHOD FOR OPERATING A PUMP DEVICE AND PUMP DEVICE
PROCÉDÉ DE FONCTIONNEMENT D'UN DISPOSITIF DE POMPE ET DISPOSITIF DE POMPE

(30) Priorität: 22.10.2013 EP 13189738
(43) Veröffentlichungstag der Anmeldung: 31.08.2016
(73) Patentinhaber: Berlin Heart GmbH, 12247 Berlin (DE)
(72) Erfinder: WIESENER, Constantin, 14471 Potsdam (DE); KARCH, Dominik, 12157 Berlin (DE)
(74) Vertreter: Pfenning, Meinig & Partner mbB
(86) Internationale Anmeldenummer: PCT/EP2014/072577
(87) Internationale Veröffentlichungsnummer: WO 2015/059158

(56) Entgegenhaltungen:
- WO-A1-97/42414
- WO-A2-2013/119752
- US-A1- 2006 052 737
- US-A1- 2010 268 334

## Beschreibung

Die Erfindung liegt auf dem Gebiet des Maschinenbaus, spezieller der Feinwerktechnik und der Elektrotechnik, und ist mit besonderem Vorteil im Bereich der Medizintechnik einsetzbar.

Konkret ist die Erfindung beispielsweise auf eine Pumpeneinrichtung mit einer Pumpe zum Ansaugen eines Fluids in einem Hohlraum eines Patientenkörpers anwendbar (siehe z.B. US2006/0052737 A). Es sind daneben jedoch auch Anwendungen außerhalb des medizinischen Gebiets denkbar, dort wo Fluide mittels einer Pumpeneinrichtung aus einem Hohlraum abgesaugt werden sollen.

Insbesondere im medizinischen Sektor stellen Pumpen zum Fördern von Fluiden in einem Patientenkörper hohe technische Anforderungen. In diesem Zusammenhang sind beispielsweise Blutpumpen bekannt, die an einer ersten Stelle im Blutgefäßsystem eines Patienten Blut ansaugen und dieses zu einer zweiten Stelle innerhalb oder außerhalb des Blutgefäßsystems fördern.

Zu diesem Zweck wird eine Pumpe, die einerseits innerhalb des Patientenkörpers, andererseits jedoch auch außerhalb des Patientenkörpers angeordnet sein kann, mit einem Katheter verbunden, der sich wenigstens teilweise im Blutgefäßsystem des Patienten befindet und dort eine Ansaugöffnung aufweist. Beispielsweise kann ein solcher Katheter eine Ansaugöffnung in einer Herzkammer eines Patienten aufweisen.

Ein bekanntes Problem beim Ansaugen von Blut besteht darin, dass ein derartiger Katheter sich durch den herrschenden Unterdruck an einer Gefäßwand festsaugen kann, wodurch die Ansaugöffnung verschlossen und das weitere Ansaugen von Blut unmöglich gemacht wird. Zudem kann dabei die Gefäßwand nachhaltig beschädigt werden.

Aus dem Stand der Technik ist bekannt, beispielsweise Pigtail-Enden im Bereich der Ansaugöffnung eines Katheters anzuordnen, um auf mechanische Weise einen Mindestabstand zwischen einer Ansaugöffnung und einer Gefäßwand zu etablieren. Dennoch lässt sich einerseits der Kontakt mit Gefäßwänden durch diese Maßnahme nicht vollständig verhindern, und andererseits ist es die Bestimmung eines solchen Pigtails, dauernd an Gefäßwände zu stoßen, was ebenfalls die Gefahr von Gewebeirritationen mit sich bringt.

Der vorliegenden Erfindung liegt vor dem Hintergrund des Standes der Technik somit die Aufgabe zugrunde, ein Verfahren zum Betrieb einer Pumpeneinrichtung und eine entsprechende Pumpeneinrichtung zu schaffen, die die geschilderten Probleme vermeidet und das dauerhafte und zuverlässige Ansaugen eines Fluids über eine Ansaugöffnung ermöglicht.

Die Aufgabe wird mit den Merkmalen der Erfindung gemäß Patentanspruch 1 bezüglich eines Verfahrens zum Betrieb einer Pumpeneinrichtung und gemäß Patentanspruch 10 bezüglich einer Pumpeneinrichtung gelöst.

Dementsprechend bezieht sich die Erfindung auf ein Verfahren zum Betrieb einer Pumpeneinrichtung, die wenigstens eine Pumpe sowie ein mit dieser verbundenes Ansaugelement mit einer Ansaugöffnung aufweist und in einem Hohlraum, insbesondere eines Patientenkörpers, ein Fluid durch Erzeugen eines Unterdrucks im Ansaugelement ansaugt, wobei während des Betriebs der Pumpeneinrichtung eine Beschleunigung gemessen und überwacht wird. Die Lösung der Aufgabe wird dadurch erreicht, dass bei Auftreten einer Beschleunigungsgröße oder einer von dieser abgeleiteten Größe, die oberhalb eines festgelegten Schwellwertes liegt, der Unterdruck im Ansaugelement zumindest für einen begrenzten Reaktionszeitraum verringert wird.

Der Erfindung liegt die Erkenntnis zugrunde, dass das Festsaugen eines Ansaugelementes mit einer Ansaugöffnung an einer Gefäßwand oder allgemein an einer Wand eines Hohlraums oft dadurch geschieht, dass das Gefäß, das den Hohlraum bildet, also beispielsweise ein Patientenkörper oder ein sonstiger Körper mit einem Hohlraum, beschleunigt wird und hierdurch Teile der Pumpeneinrichtung, also insbesondere das Ansaugelement, sofern es nicht gegenüber den Wänden des Hohlraums fixiert ist, eine Relativbewegung zu den Wänden des Hohlraums ausführen. Anders ausgedrückt bewegen sich bei einer Beschleunigung des den Hohlraum enthaltenden Gefäßes Teile der Pumpeneinrichtung im Hohlraum und können dabei leicht an die Wände des Hohlraums anstoßen, wodurch die Gefahr eines Festsaugens besteht.

Diese Gefahr kann erheblich verringert werden, indem beim Auftreten bestimmter Beschleunigungsbedingungen der Unterdruck im Ansaugelement zumindest zeitweise abgesenkt wird. Sobald die Bewegung zur Ruhe oder in einen stabilen Zustand gekommen ist, kann der Ansaugdruck wieder angehoben werden.

Grundsätzlich ist aus dem Stand der Technik eine Steuerung einer rotierenden Blutpumpe in Abhängigkeit von einer Beschleunigungsgröße bekannt. In der US-Patentanmeldung US 2008/0183287 ist ein System beschrieben, bei dem physische Aktivität eines Patienten durch Beschleunigungssensoren überwacht und in Abhängigkeit von Beschleunigungsgrößen die Drehzahl der Blutpumpe gesteuert wird. Dort richtet sich die Steuerung auf den Zweck, bei erhöhter körperlicher Aktivität eines Patienten die Unterstützung des Herzens durch eine Blutpumpe zu verstärken und somit sinnvollerweise beim Auftreten dauerhaft höherer Beschleunigungen die Pumpleistung zu erhöhen.

Die vorliegende Erfindung richtet im Gegensatz dazu nicht darauf, eine physiologische Belastung eines Patientenkörpers durch Beschleunigungsmessung zu detektieren, sondern lediglich darauf, punktuelle, stoßartige Beschleunigungen durch einzelne, ruckartige Bewegungen festzustellen, die eine im Extremfall einmalige Auslenkung von Teilen der Pumpeneinrichtung im Patientenkörper zur Folge haben können, wodurch ein Ansaugelement mit einer Ansaugöffnung an eine Gefäßwand oder eine Innenwand einer Herzkammer anstoßen könnte, wodurch das Festsaugen an dieser Wand riskiert würde. Ein dauerhaftes Erhöhen der Pumpleistung bei erhöhter körperlicher Belastung mit überdurchschnittlichen, aber nicht maximalen Beschleunigungswerten ist hierdurch nicht ausgeschlossen.

Unter stoßartigen, punktuellen Beschleunigungen werden hierbei kurzzeitige Beschleunigungen verstanden, welche beispielsweise bei Aufstehvorgängen auftreten können. In manchen Ausführungsformen kann die kurze Zeit dabei weniger als 10 sec, vorzugsweise weniger als 5 sec und besonders vorzugsweise kürzer als 2 sec auftreten und dabei innerhalb dieses Zeitrahmens eine Schwelle von betragsmäßig beispielsweise mindestens 0,5 m/s², oder beispielsweise 1 m/s² überschreiten. Als Beschleunigungsratenschwelle kann beispielsweise eine Beschleunigungsrate von mehr als 1m/s² pro sec, vorzugsweise von mehr als 3m/s² pro sec oder 5m/s² herangezogen werden. In einigen Ausführungsbeispielen werden neben den gemessenen Beschleunigungssignale weitere Größen, wie beispielsweise eine momentane relative Neigung der Pumpeneinheit zur Gravitation, erfasst und zur Klassifizierung, ob eine relevante punktuelle oder stoßartige Beschleunigung vorlag, herangezogen.

In manchen Ausführungsformen werden die Beschleunigungssignale gefiltert und beispielsweise hochfrequente Komponenten herausgefiltert. Unter hochfrequenten Komponenten werden hierbei beispielsweise Komponenten von mehr als 100 Hz, vorzugsweise mehr als 80 Hz, besonders vorzugsweise mehr als 60 Hz mittels eines Filters entfernt, d.h. die Cut-Off Frequenz des Filters liegt bei den oben genannten Werten. Ein Filter kann dabei in einer Steuervorrichtung als Schaltkreis oder als Soft- oder Firmware implementiert sein.

Die Erfindung kann außer auf medizinischem Gebiet allerdings auch in Vorrichtungen verwendet werden, bei denen ein Fluid beispielsweise aus einem Fluidtank mittels einer Ansaugleitung oder eines sonstigen Ansaugelementes abgesaugt wird. Dies kommt beispielsweise im Kraftfahrzeugbereich vor, z. B. im Wischwassertank für die Scheibenwaschanlage, wo mittels eines in einen Tank hineinragenden Schlauches Wischwasser durch eine elektrische Pumpe angesaugt wird. Wird beim Betätigen der Waschanlage das Fahrzeug beschleunigt, so kann der Ansaugschlauch sich im Tank bewegen und an einer Wand des Tanks anschlagen. Um dort ein Festsaugen zu vermeiden, kann die Pumpleistung vorübergehend verringert werden. Entsprechend ist die Erfindung beispielsweise auch beim Ansaugen von Kraftstoff aus einem Kraftstofftank anwendbar.

Als von der Beschleunigung abgeleitete Größen können auch eine Drehrate (der Pumpeneinrichtung oder des Ansaugelements) im Sinn einer Radialbeschleunigung oder die Ausrichtung einer festgelegten Achsenrichtung des Ansaugelements relativ zur Erdbeschleunigung dienen.

Eine vorteilhafte Ausgestaltung der Erfindung sieht vor, dass die Pumpleistung für einen begrenzten Reaktionszeitraum verringert wird. Durch das Absenken der Pumpleistung wird unmittelbar der Ansaugdruck im Ansaugelement verringert. Unter dem Ansaugdruck ist im Zusammenhang der vorliegenden Anmeldung die Druckdifferenz zwischen dem Fluiddruck im Ansaugelement und dem das Ansaugelement umgebenden Medium zu verstehen, wobei der Ansaugdruck derart definiert ist, dass er positiv ist, wenn der absolute hydrostatische Druck im Ansaugelement geringer ist als der hydrostatische Druck in der Umgebung des Ansaugelementes. Eine Verringerung des Ansaugdrucks bedeutet in diesem Zusammenhang eine Verringerung des Druckgefälles, d. h. des Druckunterschiedes zwischen dem Druck im Ansaugelement und dem Druck in der Umgebung des Ansaugelementes. Je kleiner der Ansaugdruck in dieser Definition ist, umso weniger schnell und kräftig wird das Fluid durch die Ansaugöffnung in das Ansaugelement gesaugt.

Vorteilhaft ausgestaltet werden kann die Erfindung zudem dadurch, dass bei Verwendung einer Rotationspumpe deren Drehzahl für einen begrenzten Reaktionszeitraum verringert wird. Eine Rotationspumpe lässt sich schnell bezüglich der Pumpleistung ansteuern, und die Pumpleistung ist stufenlos regelbar. Die Regelung der Pumpleistung kann durch die Regelung der elektrischen Leistung eines elektrischen Antriebsmotors des Rotors geschehen oder durch die Regelung der Leistung einer Mikroturbine, die die Pumpe antreibt, oder auch über die Verstellung von Rotorschaufeln oder von Leitschaufeln, die im Gehäuse der Pumpe fest installiert sind.

Unter der Reaktionszeit, für die die Pumpleistung abgesenkt wird, wird üblicherweise ein Zeitintervall verstanden, das in seiner Länge von Anfang an festgelegt sein kann. Dabei kann der Reaktionszeitraum für alle auftretenden Fälle, in denen der Ansaugdruck abgesenkt werden soll, gleich festgelegt sein, oder er kann auch von den Bedingungen abhängen, die zu einer Absenkung des Ansaugdrucks führen, d. h. von den gemessenen Beschleunigungsgrößen. Nach Ablauf des Reaktionszeitraums wird üblicherweise der Ansaugdruck wieder erhöht, was beispielsweise durch eine Erhöhung der Pumpleistung geschehen kann. Sind in der Zwischenzeit ein weiteres Mal die Bedingungen für eine Absenkung des Ansaugdrucks erfüllt, so kann der Reaktionszeitraum auch um einen weiteren Reaktionszeitraum verlängert werden. Als Reaktionszeit kann beispielsweise ein Zeitinterval von weniger als 20 sec, weniger als 10 sec, oder weniger als 5 sec verstanden werden. Die Länge des Zeitintervals des Absenkens der Pumpleistung hängt unter anderem von der gemessenen punktuellen, stoßartigen Beschleunigung ab. Dabei wird die Pumpleistung vorzugsweise direkt im Anschluss des Erfassen der punktuellen, stoßartigen Beschleunigung abgesenkt, um einen Ansaugeffekt zu vermeiden.

Eine Verringerung des Ansaugdrucks kann beispielsweise definiert sein durch eine Absenkung um wenigstens 30 % oder 50 % oder 70 % oder um eine Absenkung der Pumpleistung um wenigstens die entsprechenden Prozentbeträge. Diese Absenkungsbeträge können auch zur Definition des Beginns und Endes des Reaktionszeitraums dienen.

Eine weitere Möglichkeit zu einer vorteilhaften Ausgestaltung der Erfindung sieht vor, dass ein Ventil im Ansaugelement wenigstens für einen begrenzten Reaktionszeitraum wenigstens teilweise geschlossen wird. Auch durch das Schließen eines Ventils im Ansaugelement kann der Ansaugdruck vorübergehend verringert werden, so dass ein Festsaugen des Ansaugelementes verhindert werden kann.

Eine weitere vorteilhafte Ausgestaltung der Erfindung sieht vor, dass die Verringerung des Ansaugdrucks und/oder die Verringerung der Pumpleistung und/oder die Schließung des Ventils im Ansaugelement zu Beginn des Reaktionszeitraums schneller bewirkt wird als die Erhöhung des Ansaugdrucks und/oder die Vergrößerung der Pumpleistung und/oder das Öffnen des Ventils zum Ende des Reaktionszeitraums.

Dieser Ausgestaltung liegt der Gedanke zugrunde, dass bei Auftreten der entsprechenden Beschleunigungsbedingungen, die eine Relativbewegung des Ansaugelementes gegenüber Wänden eines Hohlraums, aus dem Fluid abgesaugt werden soll, befürchten lassen, ein sehr schnelles Absenken des Ansaugdruckes notwendig ist, bevor das Ansaugelement an der jeweiligen Wand anschlägt. Hat sich der Zustand nach der Beschleunigung normalisiert, so kann der Ansaugdruck durch entsprechende Steuerung wieder erhöht werden, ohne dass es einer besonderen Eile bedarf. Im Gegenteil kann eine mäßig langsame Erhöhung des Ansaugdrucks das Risiko verringern, dass durch einen Ruck, der durch schlagartiges Erhöhen des Ansaugdrucks entstehen könnte, das Ansaugelement ungewollt in Bewegung versetzt wird. Auch durch eine solche Bewegung könnte das Risiko eines Festsaugens des Ansaugelementes erhöht werden.

Eine weitere vorteilhafte Ausgestaltung der Erfindung sieht vor, dass als Beschleunigungsgröße, die auf Überschreiten einer Schwelle überwacht wird, die maximale punktuelle Beschleunigung oder der Ruck in Form der zeitlichen Ableitung der Beschleunigung oder eine Drehrate oder eine relative Ausrichtung der Erdbeschleunigung zu einer festgelegten Achse des Ansaugelementes dient. Die Maximalbeschleunigung, die beispielsweise unmittelbar an einem Patientenkörper oder an einer Einrichtung gemessen wird, die einen Hohlraum aufweist, aus dem ein Fluid abgesaugt werden soll, kann für die Auslenkung des Ansaugelementes in dem Hohlraum und damit für das Risiko eines Festsaugens entscheidend sein. Üblicherweise wirken elastische Rück-stellkräfte auf das Ansaugelement, die nach Ende der Beschleunigung eine Rückstellung des Ansaugelementes in eine nahezu neutrale Position bewirken. Ein weiterer Effekt, der ein erhöhtes Risiko einer Verschiebung des Ansaugelementes gegenüber den Wänden des Hohlraums mit sich bringt, ist eine plötzliche Änderung der Beschleunigung, also ein Ruck. Ein solcher Ruck bewirkt besonders große Auslenkungen des Ansaugelementes bei einer Bewegung des Körpers.

Gemäß der Erfindung wird die gemessene Beschleunigung auf das Überschreiten einer bestimmten festgesetzten Schwelle überwacht, oder es wird die Beschleunigung gemessen und laufend eine zeitliche Ableitung der Beschleunigung ermittelt, um den Ruck auf Überschreiten einer bestimmten Schwelle überwachen zu können.

Die Erfindung kann zudem vorteilhaft dadurch ausgestaltet werden, dass als Beschleunigungsgröße, die auf Überschreiten einer Schwelle überwacht wird, ein zeitliches Integral der Beschleunigung in einem festgelegten Zeitintervall dient. Dabei kann das Zeitintervall, über das die Beschleunigung zeitlich integriert wird, als rollendes Zeitintervall ständig aktualisiert werden. Dadurch werden kurze Beschleunigungsstöße weniger stark berücksichtigt als Beschleunigungen, die über einen bestimmten Mindestzeitraum andauern.

Die Beschleunigung kann mittels eines Beschleunigungssensors an einem Teil des Patientenkörpers in einer oder mehreren Richtungen gemessen werden oder direkt mittels eines Sensors an der Pumpe oder an einer Ansteuereinheit der Pumpe. Die Beschleunigung kann jeweils in einer oder mehreren Richtungen gemessen werden.

Eine Beschleunigung des Patientenkörpers lässt sich besonders einfach dadurch messen, dass am Patienten außen ein Beschleunigungssensor einfach fixiert wird. Vorteilhaft wird ein solcher Beschleunigungssensor am Rumpf des Patienten und nicht an einer Extremität befestigt.

Besonders praktisch ist es, wenn der oder die Beschleunigungssensoren unmittelbar an der Pumpe oder einer Ansteuereinheit der Pumpe befestigt werden können, so dass auch unmittelbar die Beschleunigung der Pumpe oder der Ansteuereinheit überwacht wird. Dabei wird im medizinischen Bereich davon ausgegangen, dass die Pumpe fest in den Patientenkörper integriert oder an diesem befestigt ist, so dass die Pumpe unmittelbar den Bewegungen des Körpers folgt. Das Ansaugelement dagegen hängt nur teilweise fixiert in den Hohlraum hinein und kann sich in diesem beim Auftreten von Beschleunigungen begrenzt frei bewegen.

Eine Fixierung entsprechender Beschleunigungssensoren unmittelbar an der Pumpe oder einer Ansteuereinheit der Pumpe bringt den Vorteil mit sich, dass der Aufwand bei der Installation an einem Patientenkörper verringert wird und dass die Gesamtpumpvorrichtung geschlossen vormontiert werden kann, einschließlich der Beschleunigungssensoren.

Die Erfindung bezieht sich außer auf ein Betriebsverfahren der oben beschriebenen Art auch auf eine Pumpeneinrichtung mit wenigstens einer Pumpe sowie einem mit dieser verbundenen Ansaugelement mit einer Ansaugöffnung, wobei die Pumpe dazu eingerichtet ist, durch Erzeugen eines Unterdrucks im Ansaugelement in einem Hohlraum, insbesondere eines Patientenkörpers, ein Fluid anzusaugen, wobei die Pumpeneinrichtung zudem eine Beschleunigungsmesseinrichtung mit einem Beschleunigungssensor und einer ersten Verarbeitungseinrichtung aufweist sowie eine Steuereinrichtung, die mit der ersten Verarbeitungseinrichtung verbunden ist und aufgrund von Signalen der ersten Verarbeitungseinrichtung den Unterdruck im Ansaugelement steuert, sowie mit einer zweiten Verarbeitungseinrichtung, die in Abhängigkeit von Signalen der ersten Verarbeitungseinrichtung jeweils für einen begrenzten Reaktionszeitraum den Unterdruck im Ansaugelement verringert.

Beispielsweise kann die erste Verarbeitungseinrichtung die gemessene oder laufend ermittelte Beschleunigungsgröße überwachen und das Überschreiten einer Schwelle detektieren. Die erste Verarbeitungseinrichtung kann hierzu beispielsweise auch laufend Beschleunigungswerte über die Zeit integrieren und das Integral über einen rollenden Zeitraum ebenfalls auf das Überschreiten von Schwellwerten überwachen.

Die erste Verarbeitungseinrichtung kann dann, sobald eine Schwellwertüberschreitung auftritt, ein Signal an die zweite Verarbeitungseinrichtung abgeben, die die Ansteuerung einer Pumpe oder eines Ventils beeinflusst und für den Reaktionszeitraum eine Verringerung des Ansaugdrucks bewirkt.

Im Folgenden wird die Erfindung anhand eines Ausführungsbeispiels in einer Zeichnung gezeigt und nachfolgend erläutert. Dabei zeigt
- Fig. 1: schematisch die Darstellung eines Patientenkörpers mit einer Herzkatheterpumpe, die über die Aorta in eine Herzkammer eingeführt ist,
- Fig. 2: in vergrößerter Darstellung einen durch einen Aortenbogen eingeführten Herzkatheter mit einer Herzpumpe,
- Fig. 3: zeitaufgelöst die Messdaten eines Beschleunigungssensors und die Reaktion in Form der Beeinflussung des Ansaugdrucks,
- Fig. 4: schematisch einen Steuerungs- und Datenverarbeitungsteil der Pumpeneinrichtung sowie
- Fig. 5: schematisch die Darstellung einer Rotationspumpe.

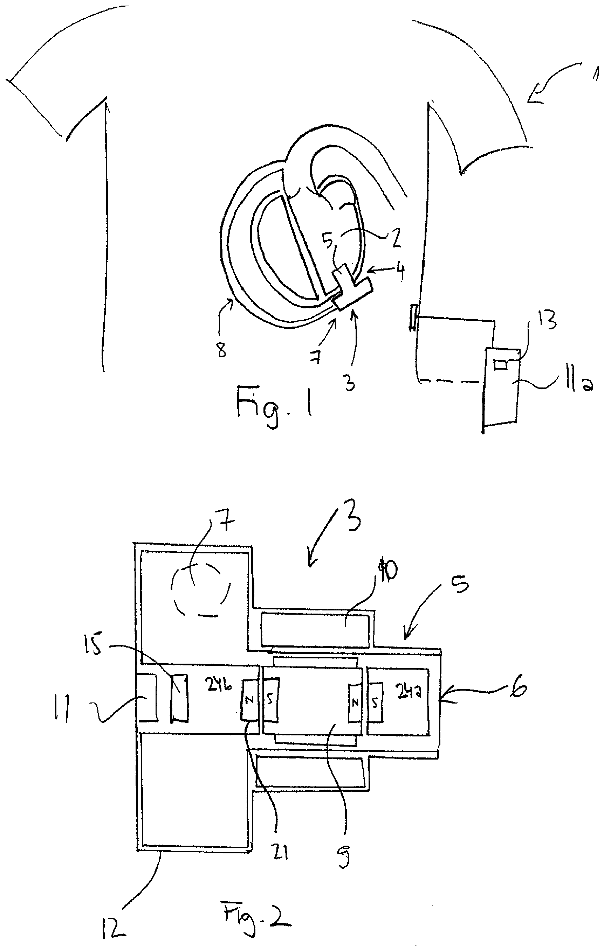

Figur 1 zeigt schematisch einen Patientenkörper 1 mit einer Herzkammer 2, in welche eine Blutpumpe 3 eingeführt ist. Die Herzpumpe ist dabei mit dem Apex 4 der Herzkammer (in diesem Fall dem linken Ventrikel des Herzens, d.h. die Pumpe ist eine left ventricular assist device oder auch LVAD) verbunden. In der Figur 2 ist die Blutpumpe 3 vergrößertdargestellt.

Ein Einlassstutzen 5 der Blutpumpe welcher direkt in den Apex eingeführt ist, weist im vorliegenden Ausführungsbeispiel eine Ansaugöffnung 6 auf, durch welche das Blut eingesaugt wird. Das Blut wird im vorliegenden Ausführungsbeispiel mittels einer axialen Blutpumpe mit einem tangentialen Auslass 7 gefördert. Der Auslass 7 ist mittels einer Kanüle 8 oder einem schlauchartigen Objekt mit der Aorta verbunden. Alternativ kann der Auslass mit der Pulmonararterie (bei einer RVAD) oder der Schulterarterie verbunden werden. Im Einlassstutzen befindet sich ein mit einer Wendel versehener Rotor 9, welcher über einen Stator 10 der Blutpumpe in Rotation versetzt werden kann und so das Blut in die Ansaugöffnung saugt. Ein Beispiel einer derartigen Pumpe ist der WO2012149946, WO2011054545, oder der WO2012150045 entnehmbar, deren Offenbarung durch Referenzieren vollumfänglich in diese Anmeldung aufgenommen ist.

Weitere Beispiele geeigneter Blutpumpen sind die Blutpumpen der EXCOR® bzw. INCOR ® Baureihe der Berlin Heart GmbH, Berlin, Deutschland. Bei der Verwendung dieser Blutpumpen werden die Pumpen über Kanülen mit dem Herzen verbunden, so dass das Ansaugelement sowohl der Einlass der Pumpe selbst als auch der Einlass der Kanüle, welche die Pumpe mit dem Herzen verbindet, sein kann.

Die Blutpumpe umfasst eine Steuervorrichtung 11, welche beispielsweise innerhalb des Pumpengehäuses 12 oder in einem zusätzlichen, im oder außerhalb des Körpers angeordneten Gehäuse (beispielsweise einer Kontrolleinheit) angeordnet sein kann. Die Steuervorrichtung ist mit mindestens einem Beschleunigungssensor verbunden (drahtlos oder drahtgebunden). Der Beschleunigungssensor ist derart konfiguriert, dass dieser eine Beschleunigung in einer oder mehreren Richtungen misst. Im gezeigten Beispiel sind drei Beschleunigungssensoren dargestellt, obgleich auch ein Beschleunigungssensor ausreichend sein kann.

Der Beschleunigungssensor 13 ist in einer externen Kontrolleinheit angeordnet, welche direkt am Körper getragen wird. Beschleunigungssensor 14 ist am Körper oder subkutan angeordnet und kommuniziert mit der Steuerungsvorrichtung über eine drahtlose Verbindung. Beschleunigungssensor 15 ist innerhalb des Pumpengehäuses angeordnet, und kann dabei in einer Variante innerhalb des blutdurchflossenen Teil der Blutpumpe angeordnet sein oder in einer weiteren Variante in einem Teil der Blutpumpe angeordnet sein, welcher nicht mit dem geförderten Blut in Berührung kommt, d.h. in dieser Variante kann der Beschleunigungssensor beispielsweise innerhalb des Statorgehäuses oder am Außenbereich des Gehäuses angeordnet sein. Die verschiedenen Orte an welchen ein Beschleunigungssensor angeordnet sein kann haben jeweils unterschiedliche Vor- bzw. Nachteile gegenüber einander.

Am distalen Ende der Blutpumpe ist der Einlassstutzen 5 als Ansaugelement dargestellt. In einigen Ausführungsbeispielen kann die Ansaugöffnung mit einem fluiddurchlässigen Gitter abgedeckt sein kann.

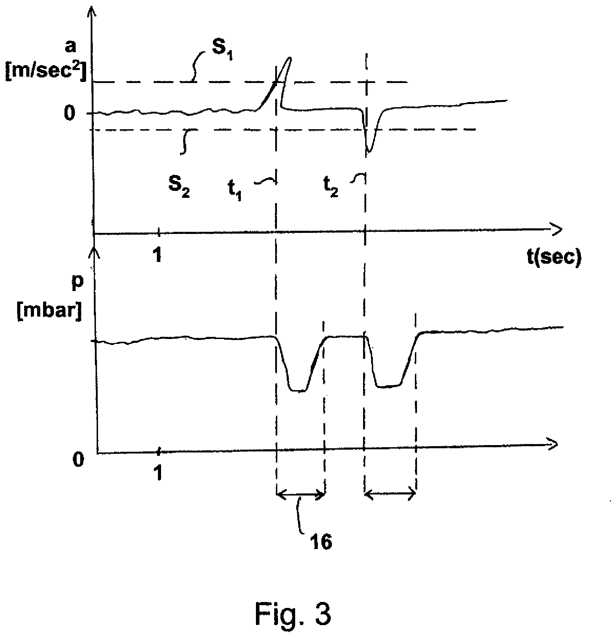

Figur 3 zeigt ein Diagramm, bei dem auf der x-Achse die Zeit in Sekunden aufgetragen ist, während im oberen Teil des Diagramms auf der y-Achse eine Beschleunigung dargestellt ist. Es sind zwei vertikale gestrichelte Linien t₁, t₂ dargestellt, von denen der Zeitpunkt t₁ dem ersten Zeitpunkt entspricht, zu dem die überwachte Beschleunigung einen Schwellwert s₁ überschreitet. Im unteren Teil des Diagramms ist deutlich, dass der Ansaugdruck, der auf der y-Achse aufgetragen und mit p benannt ist, bis zum Zeitpunkt t₁ konstant ist. Die Ansteuereinrichtung der Pumpeneinrichtung bewirkt, dass bei Überschreiten der Schwelle s₁ sofort der Ansaugdruck gesenkt wird. Dies zeigt sich im unteren Teil der Fig. 3, wo nach dem Zeitpunkt t₁ der Ansaugdruck absinkt und erst nach Verstreichen des Reaktionszeitraums 16 wieder annähernd, beispielsweise zu wenigstens 70 %, das vorherige hohe Niveau erreicht hat. Während des Reaktionszeitraums 16 wird kaum Fluid durch die Ansaugöffnung angesaugt, so dass ein Festsaugen des Ansaugelementes an einer Gefäßwand unwahrscheinlich wird.

Der Reaktionszeitraum kann entweder als Zeitraum definiert sein, während dessen der Ansaugdruck um einen Mindestprozentsatz oder einen bestimmten absoluten Wert gesenkt ist, oder als Zeitraum, der mit der Überschreitung einer Schwelle durch die überwachte Beschleunigungsgräße (zum Zeitpunkt t₁) beginnt und mit Wiederansteigen des Ansaugdrucks auf einen bestimmten Mindestprozentsatz, z. B. 70 % des Ansaugdrucks vor Beginn des Reaktionszeitraums, beendet ist.

In der Folge tritt eine Schwellenüberschreitung der zweiten Schwelle s₂ zum Zeitpunkt t₂ auf, zu dem eine Beschleunigung in einer der ersten Beschleunigung entgegengesetzten Richtung gemessen wird. Es ist sinnvoll, nur den Betrag der Beschleunigung zu überwachen, so dass ein Auslösen der Saugdruckabsenkung unabhängig von der Richtung der Beschleunigung eintritt. Im unteren Teil der Fig. 3 ist auch zu erkennen, dass nach dem Zeitpunkt t₂, zu dem eine Schwellwertüberschreitung der Beschleunigungsgröße stattfindet, für einen Reaktionszeitraum der Ansaugdruck im Ansaugelement abgesenkt wird. Hinsichtlich beispielhafter Größen oder Zeiten wird auf die vorhergehende Beschreibung verwiesen.

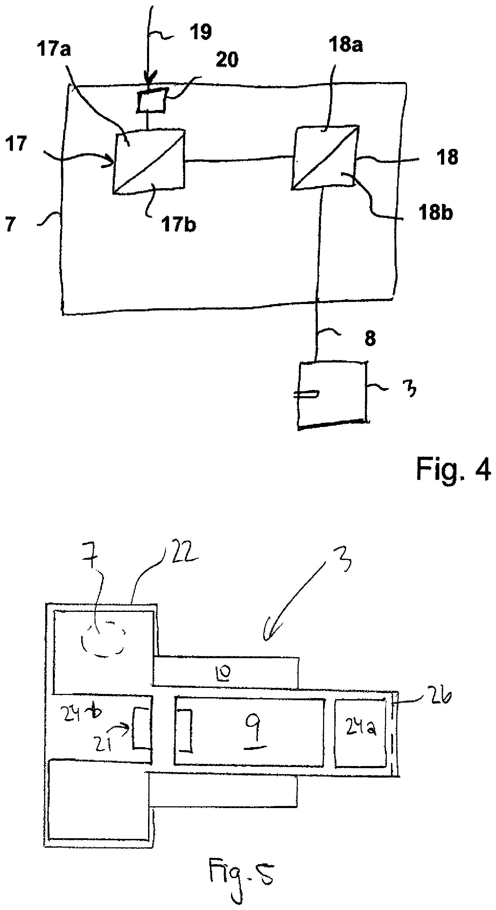

Figur 4 zeigt eine Steuereinrichtung 11a mit einer ersten Verarbeitungseinrichtung 17 und mit einer zweiten Verarbeitungseinrichtung 18. Es ist eine Sensorleitung 19 dargestellt, die die Steuereinrichtung 11a mit einem in Fig. 4 nicht dargestellten Beschleunigungssensor oder einem Gyroskop verbindet. Die Sensorleitung ist am Eingang der Steuereinrichtung 17 mit einem Digital/ Analog-Wandler 20 oder direkt mittels entsprechender Datenbusse (12C-oder SPI-Busse) mit der Steuereinrichtung verbunden, die entsprechende Beschleunigungssignale in digitalisierter Form an die erste Verarbeitungseinrichtung 17 leiten. Dort werden in einem Eingangsbereich 17a die Beschleunigungswerte laufend überwacht und gegebenenfalls komplexere Beschleunigungsgrößen, wie ein Beschleunigungsintegral über eine festgelegte Zeit oder der Ruck, laufend berechnet. In einem Vergleichsmodul 17b findet der laufende Vergleich mit einer vorgegebenen Schwelle und gegebenenfalls bei Überschreiten der Schwelle eine Signalerzeugung statt. Ein entsprechendes Signal wird der zweiten Verarbeitungseinrichtung 18 zugeleitet. Die zweite Verarbeitungseinrichtung 18 überprüft, ob der Druck bereits abgesenkt ist, weil kurz zuvor bereits eine ausreichende Beschleunigung nachgewiesen wurde, und ob aufgrund dessen der Reaktionszeitraum verlängert wird. Tritt eine Überschreitung eines Beschleunigungsschwellwertes erstmalig auf, so wird durch die zweite Verarbeitungseinrichtung 18 der den Rotor 9 und den Stator 10 aufweisenden Motor derart angesteuert, dass die Pumpleistung vorübergehend für den Reaktionszeitraum abgesenkt wird. Entsprechend wird der Speisestrom oder die Spannung des Motors verringert, so dass sich die Drehzahl des Rotors verringert. Die zweite Verarbeitungseinrichtung 18 weist dazu eine Zeitbasis 18a und eine Signalausgabe 18b auf.

Figur 5 zeigt in vergrößerter Form eine detailliertere Darstellung einer Rotationspumpe mit einem Pumpengehäuse 22, welche eine axiale Pumpe ist. Das Pumpengehäuse beherbergt einen Rotor 23, welcher zwischen einer Nabe und einer Gehäusewand oder zwei Naben 24a, 24b aktiv axial magnetisch gelagert ist, beispielsweise mittels mindestens einer Steuerspule gesteuert wird. Zudem ist der Rotor über in den Naben bzw. im Gehäuse angeordneten Magneten 21 passiv radial gelagert. Alternativ kann der Rotor auch hydrodynamisch oder durch eine Kombination aus hydrodynamischen und magntischen Lagern gelagert sein. Zur Absenkung des Ansaugdrucks im Ansaugelement kann entweder die Drehzahl des Rotors 23 gesenkt werden, oder der Rotor kann mittels eines Magnetfeldes derart verformt werden, dass seine Pumpleistung bei gleichbleibender Rotation nachlässt.

Es kann zudem jedoch auch ein Ventil vorgesehen sein, das schematisch durch die gestrichelte Linie 26 dargestellt ist. Dieses Ventil kann beispielsweise die Form einer Irisblende oder eines Klappenventils haben und jeweils von außen steuerbar sein. Das Ventil kann sowohl im Pumpengehäuse 22 als auch im Ansaugelement oder im Bereich eines Auslasselementes der Blutpumpe angeordnet sein.. Durch Schließen oder teilweises Schließen des entsprechenden Ventils ist ebenfalls eine Absenkung des Ansaugdrucks im Ansaugelement für einen Reaktionszeitraum erreichbar.

Durch die Erfindung wird ein einfaches und zuverlässiges Mittel geschaffen, um das Festsaugen eines Ansaugelementes einer Pumpe an den Wänden eines Hohlraums, aus dem ein Fluid gefördert werden soll, zu verhindern.

### Bezugszeicheniiste

- 1: Patientenkörper
- 2: Herzkammer
- 3: Blutpumpe
- 4: Apex
- 5: Einlassstutzen
- 6: Ansaugöffnung
- 7: Auslass
- 8: Kanüle
- 9: Rotor
- 10: Stator
- 11,11a: Steuerungsvorrichtung
- 12: Pumpengehäuse
- 13: erster Beschleunigungssensor
- 14: zweiter Beschleunigungssensor
- 15: dritter Beschleunigungssensor
- 16: Reaktionszeitraum
- 17: erste Verarbeitungseinrichtung
- 17a: Eingangsteil
- 17b: Vergleichsmodul
- 18: zweite Verarbeitungseinrichtung
- 18a: Zeitbasis
- 18b: Signalausgabe
- 19: Sensorleitung
- 20: Digital/Analog-Wandler
- 21: Permanentmagnete
- 22: Pumpengehäuse
- 23: Rotor
- 24a, b: Nabe
- 25: Förderelement
- 26: Ventil
- t₁, t₂: Zeitpunkte
- s₁, s₂: Beschleunigungsschwellen

## Patentansprüche

1. Verfahren zum Betrieb einer Pumpeneinrichtung, die wenigstens eine Pumpe (5) sowie ein mit dieser verbundenes Ansaugelement (12) mit einer Ansaugöffnung (6) aufweist und in einem Hohlraum (2), insbesondere eines Patientenkörpers, ein Fluid durch Erzeugen eines Unterdrucks im Ansaugelement (12) ansaugt, wobei während des Betriebs der Pumpeneinrichtung eine Beschleunigung gemessen und überwacht wird,
**dadurch gekennzeichnet, dass**
bei Auftreten einer punktuellen, stoßartigen Beschleunigungsgröße oder einer von dieser abgeleiteten Größe, die oberhalb eines festgelegten Schwellwertes (s₁, s₂) liegt, der Unterdruck im Ansaugelement (12) zumindest für einen begrenzten Reaktionszeitraum (16) verringert wird.

2. Verfahren nach Anspruch 1, **dadurch gekennzeichnet, dass** die Pumpleistung für einen begrenzten Reaktionszeitraum (16) verringert wird.

3. Verfahren nach Anspruch 2, **dadurch gekennzeichnet, dass** bei Verwendung einer Rotationspumpe (5) deren Drehzahl für einen begrenzten Reaktionszeitraum (16) verringert wird.

4. Verfahren nach Anspruch 1 oder einem der folgenden, **dadurch gekennzeichnet, dass** ein Ventil (26) im Ansaugelement wenigstens für einen begrenzten Reaktionszeitraum (16) wenigstens teilweise geschlossen wird.

5. Verfahren nach Anspruch 1 oder einem der folgenden, **dadurch gekennzeichnet, dass** die Verringerung des Ansaugdrucks und/oder die Verringerung der Pumpleistung und/oder die Schließung des Ventils (26) im Ansaugelement (12) zu Beginn des Reaktionszeitraums (16) schneller bewirkt wird als die Erhöhung des Ansaugdrucks und/oder die Vergrößerung der Pumpleistung und/oder das Öffnen des Ventils (26) zum Ende des Reaktionszeitraums.

6. Verfahren nach Anspruch 1 oder einem der folgenden, **dadurch gekennzeichnet, dass** als Beschleunigungsgröße, die auf Überschreiten einer Schwelle (s₁, s₂) überwacht wird, die maximale punktuelle Beschleunigung oder der Ruck in Form der zeitlichen Ableitung der Beschleunigung oder eine Drehrate dient.

7. Verfahren nach Anspruch 1 oder einem der folgenden, **dadurch gekennzeichnet, dass** als Beschleunigungsgröße, die auf Überschreiten einer Schwelle (s₁, s₂) überwacht wird, ein zeitliches Integral der Beschleunigung in einem festgelegten Zeitintervall dient.

8. Verfahren nach Anspruch 1 oder einem der folgenden, **dadurch gekennzeichnet, dass** die Beschleunigung eines Teils des Patientenkörpers (1) in einer oder mehreren Richtungen gemessen wird.

9. Verfahren nach Anspruch 1 oder einem der folgenden, **dadurch gekennzeichnet, dass** die Beschleunigung der Pumpe (5) oder einer Ansteuereinheit (7) der Pumpe (5) in oder am Patientenkörper (1) in einer oder mehreren Richtungen gemessen wird.

10. Pumpeneinrichtung mit wenigstens einer Pumpe (5) sowie einem mit dieser verbundenen Ansaugelement (12) mit einer Ansaugöffnung (6), wobei die Pumpe (5) dazu eingerichtet ist, durch Erzeugen eines Unterdrucks im Ansaugelement in einem Hohlraum (2), insbesondere eines Patientenkörpers, ein Fluid anzusaugen, wobei die Pumpeneinrichtung zudem eine Beschleunigungsmesseinrichtung mit einem Beschleunigungssensor (9, 10, 11) und einer ersten Verarbeitungseinrichtung (17) aufweist sowie eine Steuereinrichtung, die mit der ersten Verarbeitungseinrichtung verbunden ist und aufgrund von Signalen der ersten Verarbeitungseinrichtung den Unterdruck im Ansaugelement steuert,
**gekennzeichnet durch** eine zweite Verarbeitungseinrichtung (18), die in Abhängigkeit von Signalen der ersten Verarbeitungseinrichtung (17) jeweils für einen begrenzten Reaktionszeitraum (16) den Unterdruck im Ansaugelement (12) verringert.

## Claims

1. A method for the operation of a pump device, which comprises at least one pump (5) as well as a suction element (12), said suction element being connected to the pump and having a suction opening (6), said pump device sucking a fluid in a cavity, in particular a body of a patient, by producing a reduced pressure in the suction element (12), wherein an acceleration is measured and monitored during the operation of the pump device,
**characterised in that**
the reduced pressure in the suction element (12) is reduced at least for a limited reaction time period (16) given the occurrence of a spiked, abrupt acceleration value, or a value derived from this value, which lies above a fixed threshold value (s₁, s₂).

2. A method according to claim 1, **characterised in that** the pump power is reduced for a limited reaction time period (16).

3. A method according to claim 2, **characterised in that** in the case of the use of a rotation pump (5), its speed is reduced for a limited reaction time period (16).

4. A method according to claim 1 or one of the following ones, **characterised in that** a valve (26) in the suction element is closed at least partly, at least for a limited reaction time period (16).

5. A method according to claim 1 or one of the following ones, **characterised in that** the reduction of the suction pressure and/or the reduction of the pump power and/or the closure of the valve (26) in the suction element (12) is effected more rapidly at the beginning of the reaction time period (16) than the increase of the suction pressure and/or the increase of the pump power and/or the opening of the valve (26) towards the end of the reaction time period.

6. A method according to claim 1 or one of the following ones, **characterised in that** the maximum spiked acceleration or the jolt in the form of a temporal derivative of the acceleration, or a rotation rate serve as acceleration variables which are monitored with regard to exceeding a threshold (s₁, s₂).

7. A method according to claim 1 or one of the following ones, **characterised in that** a temporal integral of the acceleration in a fixed time interval serves as an acceleration variable which is monitored with regard to exceeding a threshold (s₁, s₂).

8. A method according to claim 1 or one of the following ones, **characterised in that** the acceleration of a part of the patient body (1) is measured in one or more directions.

9. A method according to claim 1 or one of the following ones, **characterised in that** the acceleration of the pump (5) or of an activation unit (7) of the pump (5) in or on the patient body (1) is measured in one or more directions.

10. A pump device having at least one pump (5) as well as a suction element (12), said suction element being connected to the pump and having a suction opening (6), wherein the pump (5) is configured to suck a fluid in a cavity (2), in particular of the body of a patient, by way of producing a reduced pressure in the suction element, wherein the pump device moreover comprises an acceleration measurement device having an acceleration sensor (9, 10, 11) and having a first processing device (17), as well as a control device which is connected to the first processing device and controls the reduced pressure in the suction element on the basis of the signals of the first processing device,
**characterised by** a second processing device (18) which reduces the reduced pressure in the suction element (12) for a limited reaction time period (16) depending on signals of the first processing device (17).

## Revendications

1. Procédé pour faire fonctionner un dispositif de pompage qui présente au moins une pompe (5) ainsi qu'un élément d'aspiration (12), relié avec celle-ci, avec un orifice d'aspiration (6), et dans une cavité (2), notamment un corps d'un patient, un fluide pour la génération d'une dépression dans l'élément d'aspiration (12), où, pendant le fonctionnement du dispositif de pompage, on mesure et on contrôle une accélération,
**caractérisé en ce que**
lors de l'apparition d'une grandeur d'accélération ponctuelle, saccadée ou d'une grandeur en étant dérivée qui se situe au-dessus d'une valeur de seuil (s₁, s₂) définie, la dépression dans l'élément d'aspiration (12) est diminuée au moins pour un temps de réaction (16) limité.

2. Procédé selon la revendication 1, **caractérisé en ce que** la puissance de pompage est diminuée pour un temps de réaction (16) limité.

3. Procédé selon la revendication 2, **caractérisé en ce que**, lors de l'emploi d'une pompe rotative (5), son nombre de tours est diminué pour un temps de réaction (16) limité.

4. Procédé selon la revendication 1 ou l'une des suivantes, **caractérisé en ce que** la soupape (26) dans l'élément d'aspiration est au moins partiellement fermée au moins pour un temps de réaction (16) limité.

5. Procédé selon la revendication 1 ou l'une des suivantes, **caractérisé en ce que** la diminution de la pression d'aspiration, et/ou la diminution de la puissance de pompage, et/ou la fermeture de la soupape (26) dans l'élément d'aspiration (12) au début du temps de réaction (16) est réalisée plus rapidement que l'augmentation de la pression d'aspiration, et/ou l'agrandissement de la puissance de pompage, et/ou l'ouverture de la soupape (26) à la fin du temps de réaction.

6. Procédé selon la revendication 1 ou l'une des suivantes, **caractérisé en ce qu'**en tant que grandeur d'accélération qui est contrôlée pour un dépassement d'une valeur de seuil (s₁, s₂), ou se sert de l'accélération ponctuelle maximale ou de la secousse sous la forme de la dérivée temporelle de l'accélération ou d'une vitesse rotationnelle.

7. Procédé selon la revendication 1 ou l'une des suivantes, **caractérisé en ce qu'**en tant que grandeur d'accélération qui est contrôlée en vue d'un dépassement d'une valeur de seuil (s₁, s₂), on se sert d'une intégrale temporelle de l'accélération 'dans un intervalle de temps défini.

8. Procédé selon la revendication 1 ou l'une des suivantes, caractérisé en ce l'accélération d'une partie du corps du patient (1) est mesurée dans une ou plusieurs directions.

9. Procédé selon la revendication 1 ou l'une des suivantes, **caractérisé en ce que** l'accélération de la pompe (5) ou d'une unité de démarrage (7) de la pompe (5) dans ou sur le corps du patient (1) est mesurée dans une ou plusieurs directions.

10. Dispositif de pompage avec au moins une pompe (5) ainsi qu'un élément d'aspiration (12) relié avec celle-ci, avec une orifice d'aspiration (6), où la pompe (5) est conçue pour aspirer un fluide par génération d'une dépression dans l'élément d'aspiration dans une cavité (2), notamment un corps de patient, où le dispositif de pompage présente en outre un dispositif de mesure de l'accélération avec un accéléromètre (9, 10,11) et un premier dispositif d'exploitation (17), ainsi qu'un dispositif de commande qui est relié avec le premier dispositif d'exploitation, et en raison de signaux du premier dispositif d'exploitation commande la dépression dans l'élément d'aspiration,
**caractérisé par** un deuxième dispositif d'exploitation (18), qui diminue la dépression dans l'élément d'aspiration (12) en fonction de signaux du premier dispositif d'exploitation (17) respectivement pour un temps de réaction (16) limité.
